# EUROPÄISCHE PATENTANMELDUNG

(11) **EP 0 682 910 A1**
(43) Veröffentlichungstag der Anmeldung: **22.11.1995**
(21) Anmeldenummer: 95103395.0
(22) Anmeldetag: 09.03.1995
(51) Int. Cl.: A61B 1/00

(54) **Instrument zur perkutanen Behandlung von Gewebeteilen**

(30) Priorität: 19.05.1994 DE 4417637
(71) Anmelder: Tieber, Friedrich, D-82223 Eichenau (DE); Bertagnoli, Rudolf, Dr.med., D-94360 Mitterfels (DE)
(72) Erfinder: Tieber, Friedrich, D-82223 Eichenau (DE); Bertagnoli, Rudolf, Dr.med., D-94360 Mitterfels (DE)

(57) **Zusammenfassung**

Es wird zur perutenen Behandlung von Gewebeteilen, insbesondere Bandscheiben ein Führungsinstrument (10) vorgeschlagen, das im wesentlichen aus einem Führungsrohr (12) mit einem abwinkelbaren Endstück(13) und Betätigungseinrichtungen (14, 15, 19, 22, 24) besteht, die am distalen Ende mit dem Führungsrohr verbunden sind und zum Abwinkeln des Endstückes, sowie für den Vorschub von im Rohr geführten Instrumentarien (17, 18) dienen.

## Beschreibung

Die Erfindung bezieht sich auf ein Instrument zur perkutanen Behandlung von Gewebeteilen mit einem Führungsinstrument für Instrumentarien.

Es sind Führungsinstrumente für sogenannte monoportale perkutane Behandlungen bekannt, die ein Führungsrohr enthalten, die unter Rötgenkontrolle bis zum behandelnden Gewebe geschoben werden. Durch dieses Führungsrohr wird je nach Behandlung eine Sonde, Lasersonde oder anderweitiges Instrumentarium, z.B. eine Faßzange zur Entfernung von Gewebe, geführt. Diese Behandlung erfolgt ohne direkte Sicht auf das zu behandelnde oder zu entfernende Gewebe, EP 438 241.

Abhilfe hierzu schafft das biportale Verfahren, bei dem ein zweites Führungsrohr verwendet wird, durch das die Optik bis zur Behandlungsstelle geführt wird. Hierzu ist jedoch ein zweiter Einstich erforderlich, der überdies schwierig zu handhaben ist.

Es besteht in beiden Fällen die Schwierigkeit, die Sonde oder ein Endoskop genau zu positionieren.

Der Erfindung liegt die Aufgabe zugrunde, hier Abhilfe zu schaffen, insbesondere ein Führungsinstrument zu entwickeln, mit dem das Gewebe mit Präzision und bei Bedarf kleinräumig behandelt werden kann.

Die Aufgabe wird erfindungsgemäß mit den Merkmalen des Anspruchs 1 gelöst.

Durch am Führungsinstrument vorgesehene Betätigungsmittel, mit denen die Instrumentarien präzise an den Behandlungsort geführt werden können, ist eine Behandlung sowohl qualitativ als auch zeitlich durch vereinfachte und genau einstellbare Handhabung in wesentlich verbesserter Form möglich. Diese Betätigungsmittel sind für feinjustierbare Bewegungen ausgestaltet und am äußeren Ende des Führungsintrumentes angebracht, wo sie durch Handhabung durch den Chirurgen, z.B. Drehen, Schieben von Drehknöpfen, Hebeln in eine feingradige Dreh- oder Längsbewegung des am proximalen Ende herausragenden Instrumentariums umgesetzt werden. An Skalen kann der Bewegungsgrad der Instrumentarien abgelesen werden. Mittels Fixierknöpfen werden die in die Eingriffsstellung gebrachten Instrumentarien arretiert.

Um eine exakte und während des Eingriffs stabile Plazierung der Instrumentarien zu ermöglichen, ist das Führungsinstrument mit einem Fixierrohr ausgestattet, das nach der Einführung in das Gewebe mit einer Fixationseinrichtung an einem Operationstisch fixiert wird. Das Fixierrohr dient somit als Führungs- und Bewegungsreferenz für die übrigen Instrumentarien. Das Fixierrohr kann je nach Anwendung das äußerste Führungsrohr bilden oder innerhalb eines anderweitigen äußeren Führungsrohres mittels einer Positioniereinrichtung führbar ausgebildet sein.

Gemäß einer weiteren Ausgestaltung der Erfindung ist ein Führungsrohr mit abwinkelbarem Rohrendstück vorgesehen, das mit Betätigungsmitteln abgewinkelt werden kann. Hiermit ist es möglich, die Instrumentarien, wie z.B. Sonden, Lasersonden und/oder Mikroendoskopen auch an nicht direkt erreichbare Behandlungsstellen führen zu können. Dieses ist beispielsweise von Bedeutung bei der Behandlung von Bandscheibenvorfällen, wobei nicht alle Bereiche direkt mit einer geradlinigen Sonde oder einem geradlinigen Führungsinstrument erreicht werden können. Abwinkelbare Rohre sind bereits bekannt. Eine Ausführung ist beispielsweise in der DE 42 22 121 beschrieben.

Das abwinkelbare Führungsrohr wird vorzugsweise, ausgestattet mit einer Positioniereinrichtung, innerhalb des Fixierrohres geführt, wobei es selbst zur Führung und exakten Plazierung weiterer Instrumentarien in einer Umgebung außerhalb der Instrumenten-Achslinie dient. Die proximalen Enden von Instrumentarien, wie Lasersonden, Endoskopen, Zangen, die aus dem krümmbaren Endstück des Führungsrohres herausragen, lassen sich auf diese Weise präzise dreidimensional bewegen und somit genau auf die zu behandelnde Stelle führen, auch wenn diese Stelle nicht in der Verlängerung des Führungsinstrumentes liegt. Bei Behandlungen mit Lasersonden, wie z.B. in der Nucleotomie, hat die Erfindung darüber hinaus den Vorteil, daß die Dosierung der Laserenergie aufgrund der Verkürzung des Abstandes zwischen Sonde und dem zu behandelnden Gewebe gegenüber herkömmlichen Verfahren stark reduziert werden kann. Die im Führungsrohr geführten Instrumentarien sind vorzugsweise ebenfalls mit Positioniereinrichtungen ausgestattet.

Das Führungsinstrument ist insbesondere für die perkutane Nucleotomie sowohl mit einem abwinkelbaren Rohrendstück als auch mit von Hand betätigbaren Mechanismen ausgerüstet, mit denen ein Vorschub und eine Drehung der im Führungsrohr befindlichen Instrumentarien während einer Behandlung durchführbar ist.

Dem Führungsinstrument ist gemäß einer weiteren Ausgestaltung der Erfindung ein Nucleoskop mit einem Anschlußstück für Saug- und Spülgeräte zugeordnet, so daß die verschiedenen Behandlungsvorgänge, einschließlich Ausspülen und Absaugen, in einer raschen Folge hintereinander durchführbar sind. Als Nucleoskop kann ein Fixierrohr verwendet werden, das mit entsprechender Ausgestaltung das Führungsrohr unter Beibehaltung eines Ringspaltes zur Bildung eines Flüssigkeits-Kanals umgibt.

Vorzugsweise wird für den Transport von Flüsigkeiten ein Spülkreislauf durch das Führungsinstrument verwendet. Damit wird nicht nur eine rasch durchführbare, sondern auch eine qualitativ hochwertige Spülung bzw. Absaugung der Behandlungsstelle ermöglicht. Der Spülkreislauf läßt sich fertigungstechnisch einfach durch einen Kanal innerhalb eines internen Instrumentariums, z.B. Endoskop, und einen Ringkanal realisieren, der z.B.zwischen dem Fixationsrohr oder einem internen Instrumentarium, wie dem abwinkelbaren Führungsrohr, sich befindet.

Nach einer weiteren Ausgestaltung der Erfindung ist ein Endoskop oder Mikroendoskop mit integrierter Lasersonde vorgesehen, wobei die Lasersonde getrennt vom Endoskop bewegbar ist. Eine derartige Ausführung läßt sich auch zur großräumigen Bestrahlung ausstatten. Dies kann durch eine Lasersonde mit senkrechter oder schräger Strahlenaustrittfläche erreicht werden, die exzentrisch im Endoskop angeordnet ist. Durch Rotation der Lasersonde oder des Endoskops beschreibt die Strahlenaustrittfläche unterschiedlich große Kreise, womit unterschiedlich große Strahlenfelder bewirkt werden können.

Die Erfindung wird anhand von in der Zeichnung schematisch dargestellten Ausführungsbeispielen näher beschrieben. Es zeigen:
Fig. 1 ein Instrument in seiner Gesamtheit,
Fig. 2 eine Vorschubeinrichtung,
Fig. 3 ein Datail aus Fig. 1,
Fig. 4 Feldgrößen von Laserbestrahlungen,
Fig. 5 Endoskop mit Laser-Vorschubeinrichtung und
Fig. 6 und 7 je ein weiteres Ausführungsbeispiel.

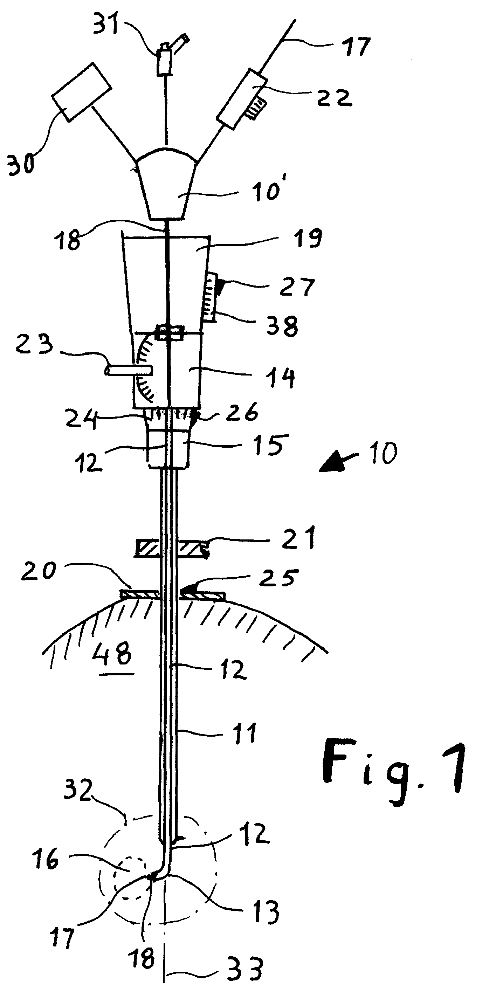

Fig. 1 zeigt eine Gesamtvorrichtung für eine monoportale perkutane Behandlung, bestehend im wesentlichen aus einem Führungsintrument 10 und einem Geräteanschluß 10'. Das Führungsinstrument 10 weist ein äußeres Fixierrohr 11 und ein darin enthaltenes abwinkelbares Führungsrohr 12 auf, dessen proximales Endstück 13 mittels einer Betätigungseinrichtung 14 bis etwa 90° abwinkelbar und mittels einer Dreheinrichtung 15 innerhalb des Fixierrohres 11 um die Längsache dreh- und fixierbar ist. Durch dieses Führungsrohr 12 werden die Instrumentarien, die für die jeweilige Behandlung erforderlich sind, bis zur Behandlungsstelle 16 geführt. In Fig. 1 sind als Instrumentarien eine Lasersonde 17 und ein Mikroendoskop 18, z.B. bekannter Bauart, gezeigt. Es können auch andere Instrumentarien, wie Zangen, Abtraggeräte, Sonden, Spüleinrichtungen und ähnliches, mit dem Führungsrohr 12 geführt und positioniert werden. Am Geräteanschluß 10' ist eine Kaltlichtquelle 30 und eine Optik 31, z.B. Videokamera, sowie eine nicht dargestellte Laserquelle anschließbar. Der Geräteanschluß 10' wird je nach Bedarf ausgebildet und mit den für den jeweiligen Eingriff notwendigen Geräten, z.B. Optikeinrichtungen, mechanische Geräte zur Betätigung von Instrumentarien, wie Bohrer, Raspeln etc., verbunden.

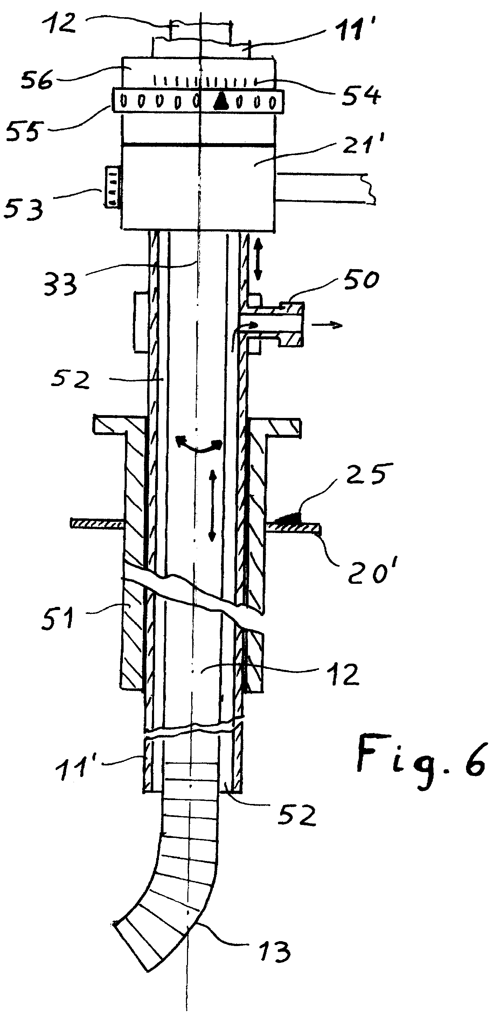

Dem Führungsinstrument 10 sind ferner Betätigungsmittel zur genauen Positionierung der durch das Führungsrohr 12 geführten Instrumentarien zugeordnet. Der Vorschub des Führungsrohres 12 erfolgt je nach Anwendung direkt manuel oder mittels einer manuell bedienbaren mechanischen Vorschubeinrichtung, wie sie beispielsweise mit dem Bezugszeichen 19 gekennzeichnet und in Fig. 5 vergrößert dargestellt ist.

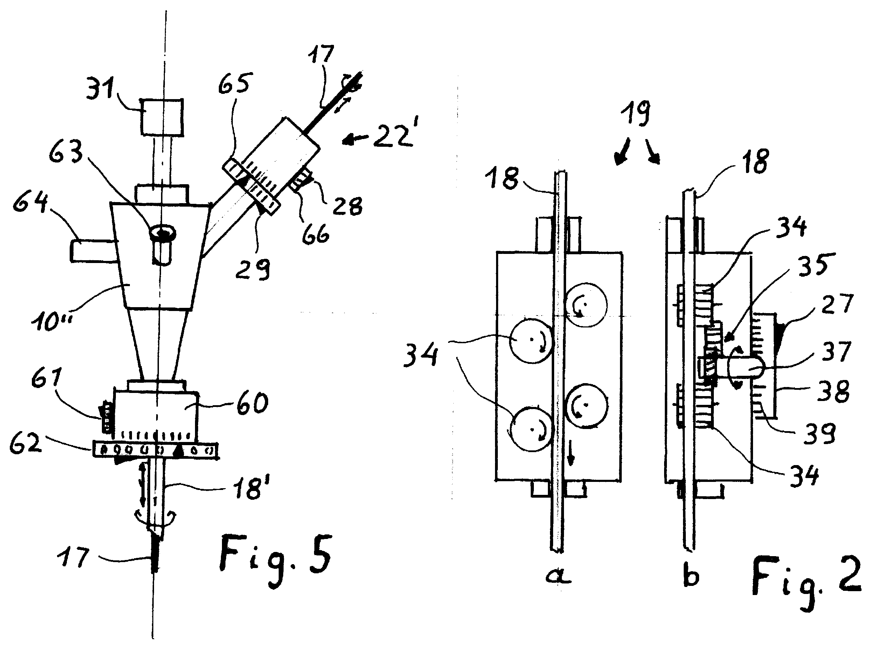

Ein Ausführungsbeispiel einer Vorschubeinrichtung 19 ist in vereinfachter Darstellung in Fig. 2 gezeigt. Die Vorschubeinrichtung 19 enthält vier Zahnwalzen 34, die über ein Zahnräderwerk 35 mit einer mit dem Zahnräderwerk 35 verbundenen Welle 37 und einem Drehring 38 in Drehverbindung stehen. Die Zahnwalzen 34 liegen mit einem entsprechenden Druck an dem präzise vorzuschiebenen Instrumentarium, z.B. dem Mikroendoskop 18, an und werden dieses bei entsprechender Drehung der Zahnwalzen 34 vor oder zurückschieben. Der Vorschub wird an einer in Verbindung mit dem Drehring 38 stehenden Skala 39 abgelesen. Fig. 2a zeigt einen Längsschnitt der Vorschubeinrichung 19 quer zu den Zahnwalzen 34. Die Fig. 2b ist ein Längsschnitt senkrecht zu Fig. 2a.

Je nach Anwendung werden dem Führungsintrument 10 ein oder mehrere Positioniereinrichtungen für verschiedene Instrumentarien und/oder Führungsrohre zugeordnet, die mittels Bajonett-, Schraub- oder anderen Verschlüssen entsprechend der jeweiligen Anwendung miteinander koppelbar sind und Teil des Führungsinstrumentes 10 bilden. Dabei sind die Instrumentarien entweder gruppenweise gemeinsam oder je getrennt innerhalb eines Führungsrohres 12 dreh-, vorschieb- oder zurückziehbar. Gemäß Fig. 1 beispielsweise ist für die Lasersonde 17 eine gesonderte Positioniereinrichtung 22 vorgesehen, mit der die Lasersonde 17 getrennt vom Mikroendoskop 18 um seine Längsachse gedreht und in Längsrichtung vorgeschoben werden kann. Es ist natürlich auch möglich, die Lasersonde 17 mit dem Mikroendoskop 18 so zu verbinden, daß beide Instrumente nur gemeinsam bewegt werden können.

Mit den manuell mittels Hebeln 23, Drehknöpfen 61,66 oder Drehringen 24,38,55,62, 65 bedienbaren Positioniereinrichtungen 14,15,19,22,54 können die Instrumentarien feinfühlig bedient und demzufolge präzise (unter röntgenografischer Beobachtung) an die vorgesehene Behandlungsstelle 16 geführt werden. Mit Fixierknöpfen 25 bis 29, 53 werden die Instrumentarien schließlich in den eingebrachten Stellungen bei Bedarf fixiert.

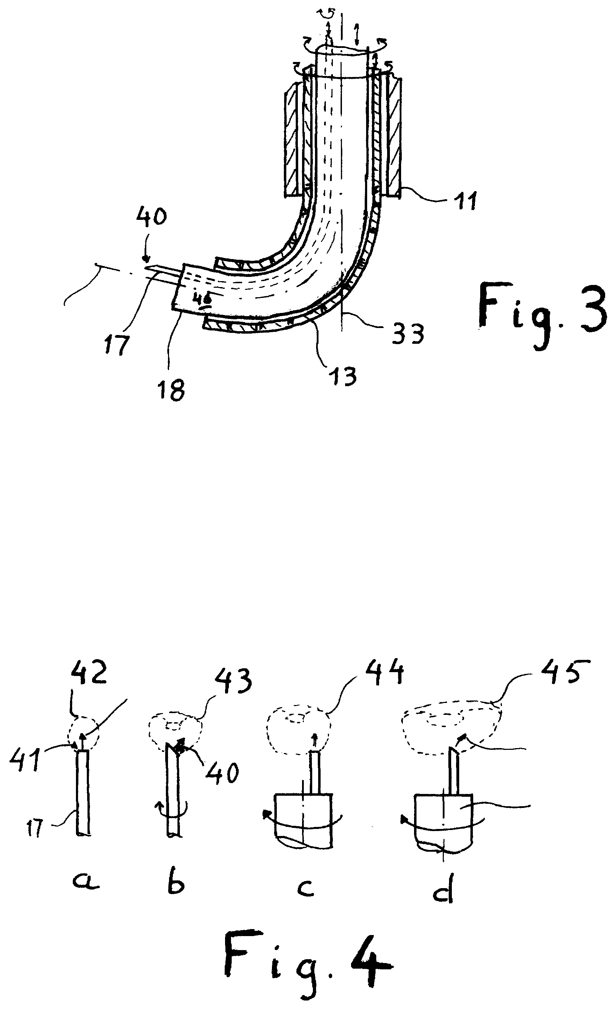

Das nach Bedarf zusammensetzbare Führungsinstrument 10 ist durch die feingradige und genaue Führbarkeit auch außerhalb der Instrumenten-Achslinie für microinvasive perkutane Behandlungen besonders gut geeignet. Die proximalen Instrumentenenden lassen sich durch Kombination von Drehbewegungen und exzentrischen Anordnungen darüber hinaus über größere Bereiche am Behandlungsort bewegen. Ein Beispiel hierzu wird nachfolgend unter Bezugnahme auf die Figuren 3 und 4 erläutert.

Fig. 3 zeigt in vergrößerter Darstellung das proximale Ende 32 (siehe Fig. 1) des Führungsinstrumentes 10. Aus dem Ende des Fixierrohres 11 ragt das in eine Krümmungsstellung gebrachte biegbare Endstück 13 des Führungsrohres 12 heraus. In diesem wird das Mikroendoskop 18 geführt, das gleichzeitig die Lasersonde 17 mit exzentrischer Anordnung trägt, die in der dargestellten Ausführung eine schräge Strahlenaustrittfläche 40 hat. Durch die Exzentrizität der Lasersonde 17 und die individuelle Bewegungmöglichkeit der beiden Instrumentarien 17 und 18 sind unterschiedlich große Feldbereiche bestrahlbar.

Fig. 4a bis 4d zeigen vier unterschiedliche, mit Laserstrahlen einwirkbare Bereiche des zu behandelnden Gewebes. Der kleinstmögliche Feldbereich 42 wird durch eine nicht bewegte Lasersonde 17 mit senkrechter Austrittsfläche 41, wie in Fig. 4a gezeigt ist, oder schräger Austrittsfläche 40 erreicht. Mit einer schrägen Austrittsfläche 40 kann der Feldbereich 43 durch Rotation der Lasersonde 17 vergrößert werden, Fig.4b.

Weitere Vergrößerungen erreicht man, wenn eine Lasersonde 17 mit quergerichteter Austrittsfläche 41 (Fig. 4c, Feldbereich 44) bzw. mit schräger Austrittsfläche 40 (Fig. 4d, Feldbereich 45) mit dem Mikroendoskop 12 um die Führungsinstrumenten-Achse 33' bewegt wird. Dabei ist der Feldbereich 45 größer als der Feldbereich 44.

In Fig. 5 ist eine Ausführung eines Mikroendoskopes 18' mit integrierter Lasersonde 17' dargestellt, mit dem unterschiedlich große Feldbereiche behandelt werden können. An einem Geräteanschluß 10'' ist eine Positioniereinrichtung 60 mit einem Drehknopf 61 für den Vorschub und einem Drehring 62 für die Rotationsbewegung des Mikroendoskops 18' verbunden. Die Optik 31 für das Mikroendoskop 18' schließt sich an der Stirnseite des Geräteanschlußes 10'' an. Schräg am Außenumfang mündet die Lasersonde 17 mit der Vorschub- und Rotationseinrichtung 22' in den Geräteanschluß 10'' ein, worin die Lasersonde 17 in das Mikroendoskop 18' eingeführt ist. In dem Mikroendoskop 18' ist ferner ein Kanal 46 (Fig. 3) zur Einführung von Lösungen, Spülmitteln, usw. vorgesehen, der mit einem Anschlußstutzen 63 am Geräteanschluß 10'' in Verbindung steht. Ferner ist ein Kaltlichtanschluß 64 angebracht.

Das äußere Fixierrohr 11 dient zur Fixierung des Führungsinstrumentes 10 am menschlichen Körper. Dazu ist eine Körperdistanzscheibe 20, die sich am Körper abstützt und mit dem durch eine zentrische Bohrung geführten Fixierrohr 11 verbindbar und mittels eines Fixierknopfes 25 arretierbar ist, und eine Fixationsvorrichtung 21 vorgesehen, über die das Fixierrohr 11 andererseits mit dem OP-Tisch befestigt wird.

Fig. 6 zeigt ein Beispiel, bei dem das Fixierrohr 11 gleichzeitig mit Präzision an die Behandlungsstelle heranführbar ist. Dieses ist beispielsweise erforderlich, wenn das Fixierrohr 11' als Nucleoscop mit einem Absaugstutzen 50 ausgebildet ist. In derartigen Fällen ist ein weiteres Rohr, nämlich ein äußeres Führungsrohr 51 erforderlich, das ggf. mit einer Körperdistanzscheibe 20 ausgerüstet ist und das zur Führung des axial verschiebbaren Fixierrohres 11' dient. Der innere Durchmesser des Fixierrohres 11' ist so groß bemessen, daß zwischen dem darin geführten krümmbaren Führungsrohr 12 oder einem anderweitigen zylindrischen Instrumentarium ein Spalt 52 verbleibt, über den abzusaugendes Fluid von der zu behandelden Stelle zum Absaugstutzen 50 gelangen kann. Mit dem Absaugstutzen wird eine nicht dargestellte Absaugeinrichtung verbunden. Der Spalt 52 läßt sich in Verbindung mit einem anderweitigen Flüssigkeitszufuhrkanal zu einem Flüssigkeitskreislauf kombinieren. Der Zufuhrkanal kann beispielsweise der im Mikroendoskop 18' vorgesehene Kanal 46 sein. Der Kanal 52 des Nucleoskops kann selbstverständlich auch als Zufuhr- und Absaugleitung benutzt werden.

Das Fixierrohr 11' ist in diesem Fall axial verschiebbar mit einer Fixationsvorrichtung 21' für den OP-Tisch verbunden und mittels eines Drehknopfes 53 in der gewählten Stellung arretierbar. Mit der Fixationsvorrichtung 21' ist eine von Hand betätigbare Vorschubeinrichtung 54 für das Fixierrohr 11' verbunden, die einen Drehring 55 mit zugehöriger Skala 56 aufweist. Der Vorschubmechanismus kann beispielsweise ein Schraube-Mutter-System mit entsprechend feinem Gewinde sein, wobei der Drehring 55 die Mutter bildet.

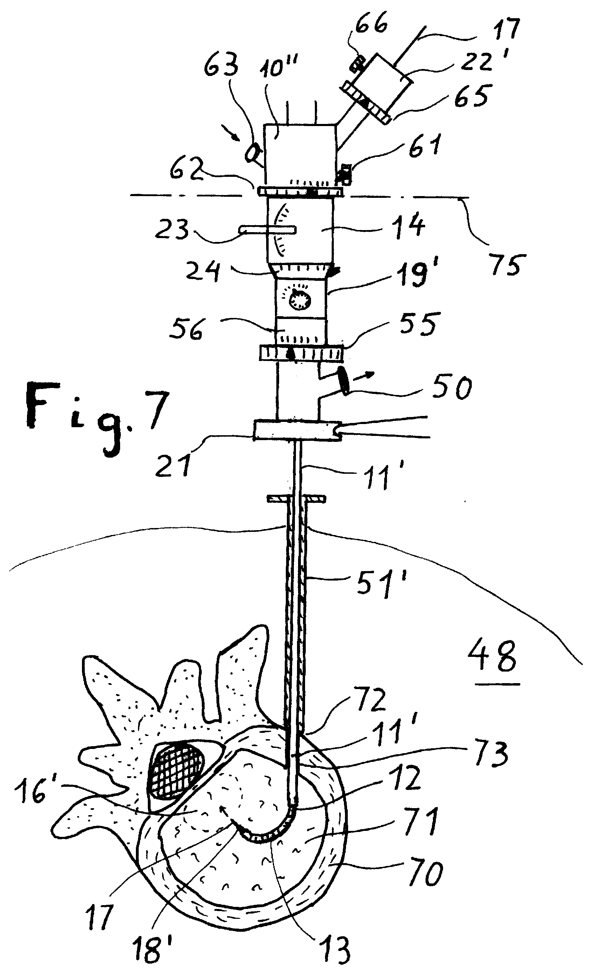

Fig. 7 zeigt eine Anwendung eines Führungsinstrumentes 10 zur Behandlung eines Bandscheibenvorfalles, wobei das Führungsintrument 10 sich im wesentlichen aus einer Kombination der in Figuren 5 und 6 dargestellten Bestandteile zusammensetzt.

Nach der Vorbereitung eines Einführkanals durch Muskelgewebe 48 mit bekannten Mitteln und Verfahren und dem Durchstoßen des Faserringes bzw. äußeren Annulus 70 wird ein äußeres Führungsrohr 51' bis zum Anschlag an den Annulus 70 von Hand eingeschoben. In das äußere Führungsrohr 51' kommt das mit der Fixationsvorrichtung 21 oder 21' und Positioniereinrichtung 56 ausgestattete Fixierrohr 11'. Mit der Positioniereinrichtung 56 wird die Eindringtiefe des Fixierrohres 11' in der Bandscheibe 71 genau eingestellt und arretiert. Anschließend wird das abwinkelbare Führungsrohr 12 mit dem darin enthaltenen Mikroendoskop 18' in das Fixierrohr 11' eingeführt, mit dem Drehring 24 so gedreht, daß die Abwinkelrichtung des gegliederten Endstücks 13 zur Behandlungsstelle 16' zeigt, mittels der Vorschubeinrichtung 19' präzise vorgeschoben und arretiert. Schließlich wird das Endstück 13 mit dem Hebel 23 gekrümmt, derart, daß der Führungsrohraustritt zur Behandlungsstelle 16' zeigt. Das Mikroendoskop 18' enthält die Lasersonde 17 entsprechend Fig.5.

In dieser Stellung ist es dann möglich, mit den Arbeitsinstrumentarien zur Behandlungsstelle zu gelangen und die Behandlung durchzuführen. Bei einem Bandscheibenvorfall wird der Bereich 16' der Bandscheibe 71 zunächst mittels Laserstrahlen aufgeweicht, wobei die Größe des zu bestrahlenden Bereiches entsprechend durch die in Verbindung mit Fig. 4 beschriebenen Möglichkeiten gewählt wird. Anschließend wird das aufgeweichte Gewebe der Bandscheibe 71 durch den Kanal 25 des Fixierrohres 11' (Fig. 6) abgesaugt.

Ein ggf. erforderlicher Spülvorgang wird über einen Kreislauf durchgeführt. D.h. das Spülmittel wird durch den Zuführstutzen 63 und über beispielsweise einen im Mikroendoskop 18' befindlichen Kanal 46 (Fig. 2) in die Behandlungsstelle eingebracht und über einen davon getrennten Kanal, z.B. den Spalt 52 (Fig. 6) zwischen dem krümmbaren Führungsrohr 12 und dem Fixationsrohr 11' wieder abgesaugt. Um einen Austritt von Spülmitteln aus dem Bandscheibenbereich in das Muskelgewebe 48 zu vermeiden, ist das freie Stirnende 72 des äußeren Führungsrohres 51' als Abdichtungselement ausgebildet, um den Durchbruch 73 im Annulus 70 gegenüber dem umgebenden Muskelgewebe 48 abzudichten.

Gemäß Fig. 7 stellt das Führungsinstrument 10 nach dem Zusammensetzen der einzelnen Komponenten (Instrumentarien, Positioniereinrichtungen) eine Baueinheit dar, wobei die verschiedenen Positioniereinrichtungen über entsprechende Verschlüße miteinander verbunden sind. Die Handhabung erfolgt in so einem Fall unmittelbar in der Nähe des Patienten. Für längere Eingriffe kann ein Teil der Positioniereinrichtungen zu einem Gerätetisch verlagert werden, um die kontrollierte Handhabung der Bedienungselemente zu erleichtern und damit ein Gelingen des Eingriffs sicherzustellen. Bei dem in Fig. 7 gezeigten Beispiel, könnte dazu das Insrumentarium an der Linie 75 getrennt werden, so daß das Mikroendoskop 18', die Lasersonde 17 und die Spülung von einen Gerätetisch aus bedient werden, wobei das flexible Mikroendoskop 18' die Verbindung zwischen den am Patienten bzw Gerätetisch befindlichen Teilen des Führungsinstruments herstellt.

Für häufig vorkommende Eingriffe können die dazu verwendeten Instrumentarien mit den zugehörigen Positioniereinrichtungen so ausgestaltet werden, daß sie vor deren Einführung in ein am Patienten befindlichen, fixierten Führungrohr zu einer kompletten Baueinheit zusammengesetzt werden können. Dadurch werden die erforderlichen Handgriffe während des Eingriffes minimieren. Verschiedene Instrumentarien können mit den zugehörigen Positioniereinrichtungen auch so ausgebildet werden, daß sie beliebig, d.h. mit entsprechend abgestimmten Verbindungselementen, zusammensetzbar sind. Außerdem ist es möglich, zumindest einen Teil der Positioniereinrichtungen nicht per Hand, sondern über eine Steuervorrichtung einzustellen. Das Steuergerät kann dabei über einzustellende Parametern oder mit Steuersignalen betrieben werden, die von einem Rechner ausgegeben werden.

Die Erfindung ist nicht auf die vorstehend beschriebenen Beispiele beschränkt, sie erstreckt sich vielmehr auf alle Ausführungsformen, mit denen die genaue Plazierung von Instrumentarien unter Anwendung eines Führungsinstrumentes möglich ist.

## Patentansprüche

1. Instrument zur perkutanen Behandlung von Gewebeteilen mit einem Führungsinstrument für Instrumentarien, dadurch gekennzeichnet, daß das Führungsinstrument (10) Betätigungsmittel (14,15,19,22,56,60) aufweist, mit denen die Instrumentarien (12,17,18,18') längs verschiebbar und/oder um deren Längsachse (33) drehbar sind und damit präzise an die Behandlungsstelle (16,16') führbar sind.

2. Instrument nach Anspruch 1, dadurch gekennzeichnet, daß das Führungsinstrument (10) zusätzlich Fixiereinrichtungen (21,21',25 - 28) aufweist, mit denen die Instrumentarien (12,17,18,18') in der eingebrachten Stellung und das Führungsinstrument (10) gegenüber einem OP-Tisch fixierbar sind.

3. Instrument nach Anspruch 1 oder 2, dadurch gekennzeichnet, daß dem Führungsinstrument (10) ein Fixationsrohr (11,11') und ein in dem Fixationsrohr mit Präzision führbares Führungsrohr (12) mit abwinkelbarem proximalen Endstück (13) zugeordnet ist.

4. Instrument nach einem der vorhergehenden Ansprüche, dadurch gekennzeichnet, daß das Führungsinstrument (10) mit einem Spülkreislauf (63,46,52,50) ausgestattet ist.

5. Instrument nach einem der vorhergenhenden Ansprüche, dadurch gekennzeichnet, daß konzentrisch ineinander geführte Instrumentarien (12,17,18,18') je gesonderte, als Positioniereinrichtungen ausgestaltete Betätigungsmittel (14,15,19,22,54,56,60) zugeordnet sind, mit denen die Instrumentarien je getrennt in Länsrichtung des Führungsinstruments (10) verschoben und/oder um die Längsachse (33) gedreht werden können.

6. Instrument nach Anspruch 1 oder 5, dadurch gekennzeichnet, daß die Betätigungsmittel (14,15,19,22,54,56,60) mit Skalen (39,54) ausgestattet und so ausgebildet sind, daß kontrollierte stufenlose Verschiebungen und Drehungen der Instrumentarien (12,17,18,18') möglich sind und/oder daß die Instrumentarien mit rasterartige Bewegungen im Millimeterbereich betätigbar sind.

7. Instrument nach Anspruch 1, 5 oder 6, dadurch gekennzeichnet, daß zumindest ein Teil der Betätigungsmittel (15,19,22',60)mit Fixierknöpfen (25-29,53) versehen sind, mit denen die Instrumentarien(12,17,18,18') in ihren ausgerichteten Positionen fixierbar sind.

8. Instrument nach einem der vorhergehenden Ansprüche, dadurch gekennzeichnet, daß als Intrumentarium ein Endoskop oder Mikroendoskop (18,18') mit integrierter Lasersonde (17) und ggf. einem Kanal (46) zur Führung von Flüssigkeiten vorgesehen ist.

9. Instrument nach Anspruch 5, dadurch gekennzeichnet, daß die Lasersonde (17) im Endoskop (18,18') exzentrisch angeordnet ist und daß dem Endoskop und der Lasersonde getrennte Betätigungsmittel (60 bzw.22') zugeordnet sind.
